# EUROPEAN PATENT APPLICATION

(11) **EP 4 585 217 A1**
(43) Date of publication of application: **16.07.2025**
(21) Application number: 23863561.9
(22) Date of filing: 08.09.2023
(51) Int. Cl.: A61K 31/7048, A61P 3/10, A61P 3/04, A61P 1/16

(54) **PHARMACEUTICAL COMPOSITION COMPRISING METABOLITE OF ENAVOGLIFLOZIN, AND USE THEREOF**

(30) Priority: 08.09.2022 KR 20220114500
(71) Applicant: Daewoong Pharmaceutical Co., Ltd., Hwaseong-si, Gyenoggi-do 18623 (KR)
(72) Inventor: PARK, Sun Hwa, Hwaseong-si Gyeonggi-do 18479 (KR); JI, Hye Young, Yongin-si Gyeonggi-do 17010 (KR); CHOI, Ji Soo, Seoul 06310 (KR); YOUN, A Hye, Seoul 03165 (KR); PARK, Mi Jie, Hwaseong-si Gyeonggi-do 18310 (KR); PARK, Joon Seok, Yongin-si Gyeonggi-do 17000 (KR)
(74) Representative: Plasseraud IP
(86) International application number: PCT/KR2023/013524
(87) International publication number: WO 2024/054097

(57) **Abstract**

The present invention relates to a pharmaceutical composition comprising a metabolite of enavogliflozin, and a use thereof. An enavogliflozin M1 metabolite of chemical formula 1 is a SGLT1/SGLT2 dual inhibitor and exhibits a different pharmacological mechanism from enavogliflozin which is a SGLT2 selective inhibitor. A pharmaceutical composition comprising, as an active ingredient, the enavogliflozin M1 metabolite that is a SGLT1/SGLT2 dual inhibitor can be useful in the prevention or treatment of diabetes or heart failure.

## Description

### [Technical Field]

The present invention relates to a pharmaceutical composition comprising a metabolite of enavogliflozin and a use thereof.

### [Background Art]

Sodium glucose cotransporter 2 (SGLT2) is a transporter that is responsible for glucose reabsorption in the kidneys together with sodium glucose cotransporter 1 (SGLT1), and SGLT2 plays most of the role. Therefore, when an SGLT2 inhibitor inhibits the SGLT2 transporter, the glucose excreted into the urine increases, which ultimately lowers blood sugar levels and furthermore, the calories contained in blood sugar are excreted, resulting in the effect of losing weight.

One of the drugs developed as an SGLT2 inhibitor that can be effectively used as a therapeutic for type 2 diabetes due to such effects is enavogliflozin, which is represented by the following structural formula (Chemical Formula A) and disclosed in Korean Patent Publication No. 2014-0022086 (Patent Document 1).

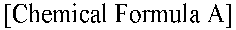

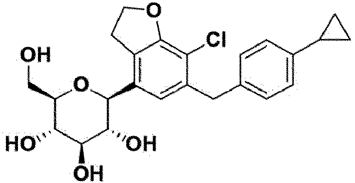

### Compound name: (2S,3R,4R,5S,6R)-2-(7-chloro-6(4-cyclopropylbenzyl)-2,3-dihydrobenzofuran-4-yl)-6-(hydroxymethyl)tetrahydro-2H-pyran -3,4,5-triol

Enavogliflozin is a drug that treats type 2 diabetes by selectively inhibiting SGLT2 and exhibits the same or better efficacy with only 0.3 mg, which is less than 1/30th of the amount of existing SGLT2 inhibitors. Through phase 3 clinical trials conducted on patients with type 2 diabetes, it was proven that enavogliflozin has superior glycated hemoglobin (HbA1c) and fasting blood sugar lowering effects and safety compared to existing commercially available drugs, and thus enavogliflozin was approved for marketing.

According to Non-Patent Document 1, enavogliflozin was confirmed to produce a total of five metabolites in human hepatocytes: M1, M2, M3, U1, and U2. The formation of M1 and M2 from enavogliflozin was catalyzed by CYP3A4 and CYP2C19. M3 was produced by hydroxylation of M1, which was catalyzed by CYP3A4. The formation of U1 was catalyzed by UGT2B7, whereas the formation of U2 was catalyzed by UGT1A4, UGT1A9, and UGT2B7.

Non-Patent Document 1 is a paper on the elucidation of the drug metabolism of enavogliflozin in the liver and the metabolites and metabolic enzymes of enavogliflozin, but it does not reveal anything about the activity exhibited by the metabolites of enavogliflozin.

### [Related Art Document]

### [Patent Document]

Korean Patent Publication No. 2014-0022086

### [Non-Patent Document]

Ju-Hyun Kim et al., Pharmaceutics. 2020 Sep 11;12(9):865. doi: 10.3390/pharmaceutics12090865.

### [Disclosure]

### [Technical Problem]

The present invention aims to provide a pharmaceutical use of a metabolite of enavogliflozin.

### [Technical Solution]

In the process of studying the metabolites of enavogliflozin, the present inventors confirmed that the enavogliflozin M1 metabolite represented by Chemical Formula 1 is a sodium glucose cotransporter 1 (SGLT1)/sodium glucose cotransporter 2 (SGLT2) dual inhibitor and exhibits a pharmacological mechanism that is different from that of enavogliflozin, which is an SGLT2 selective inhibitor, and thereby completed the present invention.

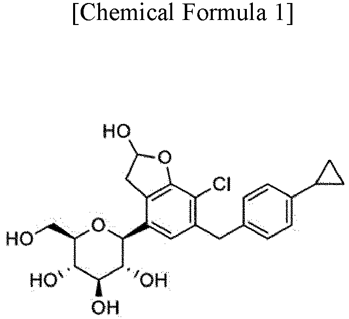

### (2S,3R,4R,5S,6R)-2-(7-chloro-6-(4-cyclopropylbenzyl)-2-hydroxy-2,3-dihydrobenzofuran-4-yl)-6-(hydroxymethyl)tetrahydro-2H-pyran-3,4,5-triol

SGLT2 inhibitors target SGLT2, which is responsible for reabsorption of more than 90% of glucose filtered by the kidneys, and SGLT1 inhibitors target SGLT1, which is responsible for reabsorption of the remaining 10% of glucose in the small intestinal epithelial cells.

Most of the SGLT2 inhibitors developed previously were drugs with high selective binding affinity for SGLT2, and enavogliflozin, developed by the applicant of the present invention, is also a drug with very high selectivity for SGLT2 compared to SGLT1, and was recently approved as a therapeutic for type 2 diabetes.

As an SGLT1/SGLT2 dual inhibitor, Lexicon Pharmaceuticals' sotagliflozin was approved for type 1 diabetes in the EU and for heart failure (HF) by the US Food and Drug Administration (FDA).

The present invention provides a use of the enavogliflozin M1 metabolite as an SGLT1/SGLT2 dual inhibitor.

Hereinafter, the present invention is described more specifically.

The present invention provides a use of the enavogliflozin M1 metabolite or a pharmaceutically acceptable salt thereof as an SGLT1/SGLT2 dual inhibitor, a pharmaceutical composition for use in preventing or treating diabetes or heart failure, comprising the enavogliflozin M1 metabolite or a pharmaceutically acceptable salt thereof as an active ingredient, and a method of preventing or treating diabetes or heart failure, comprising administering an effective amount of enavogliflozin M1 metabolite or a pharmaceutically acceptable salt thereof to a subject in need thereof.

The present invention provides a pharmaceutical composition for use in preventing or treating diabetes or heart failure, comprising the enavogliflozin M1 metabolite represented by Chemical Formula 1 or a pharmaceutically acceptable salt thereof as an active ingredient.

In addition, the present invention provides an SGLT1/SGLT2 dual inhibitor comprising the enavogliflozin M1 metabolite represented by Chemical Formula 1 or a pharmaceutically acceptable salt thereof as an active ingredient.

As can be confirmed in the examples described below, the enavogliflozin M1 metabolite has binding affinity for SGLT2 and at the same time, it also exhibits strong binding affinity for SGLT1. Therefore, the enavogliflozin M1 metabolite is a drug candidate that can act on both indications that may be treated with an SGLT2 inhibitor and indications that may be treated with an SGLT1/SGLT2 dual inhibitor.

In the present invention, the diabetes may be type 1 diabetes or type 2 diabetes.

A representative indication that may be treated with an SGLT2 inhibitor may be type 2 diabetes. In Example 3 described below, the enavogliflozin M1 metabolite was confirmed to have excellent antidiabetic efficacy through an oral glucose tolerance test (OGTT) and urinary glucose excretion (UGE) in a normal rat model.

Representative indications that may be treated with an SGLT1/SGLT2 dual inhibitor may be type 1 diabetes and heart failure.

Since the enavogliflozin M1 metabolite can simultaneously block SGLT2 present in the kidneys and SGLT1 present in the small intestine, it can effectively act on type 1 diabetes with insulin secretion disorders. In Example 4 described below, the antidiabetic efficacy of the enavogliflozin M1 metabolite in a type 1 diabetic rat model was demonstrated. Example 4 suggests that the enavogliflozin M1 metabolite may be the most effective drug for type 1 diabetes compared to enavogliflozin or sotagliflozin.

In one specific example, the pharmaceutical composition may be administered to a patient with diabetes or a patient in the pre-diabetic stage.

A pharmaceutical composition comprising the enavogliflozin M1 metabolite or a pharmaceutically acceptable salt thereof as an active ingredient may be used in the form of single administration or combined administration.

The enavogliflozin M1 metabolite alone is sufficient to prevent or treat diabetes and/or heart failure. However, since antidiabetic drugs or anti-heart failure drugs are administered in combination for various purposes and may exhibit complementary or synergistic effects when administered in combination, a pharmaceutical composition comprising the enavogliflozin M1 metabolite as an active ingredient may be administered in combination with another antidiabetic drug or anti-heart failure drug.

For example, it may be administered in combination with known antidiabetic drugs including biguanide-based drugs, such as metformin, dipeptidyl peptidase-4 (DPP4) inhibitors, sulfonylurea-based insulin secretagogues, and/or insulin.

In addition, another indication that may be treated with an SGLT1/SGLT2 dual inhibitor may be heart failure. The efficacy of sotagliflozin as an SGLT1/SGLT2 dual inhibitor in preventing or treating heart failure has been proven through US FDA approval.

The term "prevention" as used herein refers to any action that inhibits or delays the onset of diabetes or heart failure by administering the pharmaceutical composition according to the present invention.

In addition, the term "treatment" as used herein refers to any action that ameliorates or beneficially changes diabetes or heart failure by administering the pharmaceutical composition according to the present invention.

The dose of the enavogliflozin M1 metabolite, which may be used for the prevention or treatment of diabetes and/or heart failure in patients with or at risk of diabetes and/or heart failure, is not particularly limited, and may be appropriately adjusted according to the disease severity, weight, age, sex, presence of other complications of the patient, and the like.

The enavogliflozin M1 metabolite or a pharmaceutically acceptable salt thereof may be administered orally or parenterally. The administration period may be appropriately adjusted by a clinician based on the effect of preventing or treating diabetes and/or heart failure in patients with or at risk of diabetes and/or heart failure according to the administration thereof.

The pharmaceutical composition according to the present invention may further contain a pharmaceutically acceptable carrier in addition to the enavogliflozin M1 metabolite of Chemical Formula 1 or a pharmaceutically acceptable salt thereof, which is the active ingredient, and may be formulated together with the carrier.

The term "pharmaceutically acceptable carrier" as used herein refers to a carrier or diluent that does not stimulate a living organism and does not inhibit the biological activity and properties of the administered compound. Pharmaceutically acceptable carriers in a composition formulated as a liquid solution, which are sterile and biocompatible carriers, include a mixture of one or more of saline solutions, sterile water, Ringer's solution, buffered saline, albumin injection solutions, dextrose solutions, maltodextrin solutions, glycerol, ethanol, and one or more of these components. In addition, other conventional additives such as antioxidants, buffers, and bacteriostatic agents, may be added as needed. In addition, diluents, dispersants, surfactants, binders, and lubricants may be further added to formulate the composition into injectable formulations such as aqueous solutions, suspensions, and emulsions, pills, capsules, granules, or tablets.

In the present invention, the pharmaceutical composition may have a dosage form for oral administration, such as a tablet or capsule. In one specific embodiment of the present invention, the pharmaceutical composition may have the dosage form of a tablet.

When the dosage form is for oral administration, the pharmaceutical composition of the present invention may include excipients, disintegrants, and binders as additives.

Examples of excipients include lactose (including hydrates), dextrin, mannitol, sorbitol, starch, microcrystalline cellulose (e.g., Celphere^{™}), silicified microcrystalline cellulose (e.g., Prosolv^{™}), calcium phosphate hydrate, anhydrous calcium phosphate, calcium carbonate, sugars, or a mixture thereof. In a specific embodiment of the present invention, a preferred excipient is microcrystalline cellulose.

Examples of disintegrants include crospovidone, croscarmellose sodium, sodium starch glycolate, and low-substituted hydroxypropyl cellulose. In a specific embodiment of the present invention, a preferred excipient is croscarmellose sodium.

Examples of binders include polyvinylpyrrolidone, povidone, gelatin, starch, sucrose, methylcellulose, ethylcellulose, hydroxyethylcellulose, hydroxypropylcellulose, hydroxypropylalkylcellulose (e.g., hydroxypropylmethylcellulose), and a mixture thereof. In a specific embodiment of the present invention, a preferred binder is hydroxypropylcellulose.

Examples of other additives include lubricants, colorants, and the like.

The lubricants include stearic acid, stearic acid salts (e.g., magnesium stearate), light anhydrous silicic acid, talc, corn starch, carnauba wax, magnesium silicate, synthetic aluminum silicate, hydrogenated oils, white lead, titanium oxide, microcrystalline cellulose, Macrogol 4000 and 6000, isopropyl myristate, calcium hydrogen phosphate, and a mixture thereof.

In the present invention, the pharmaceutical composition may have a dosage form for parenteral administration. For example, a formulation for parenteral administration containing the composition of the present invention as an active ingredient may be formulated in an injectable form such as subcutaneous injection, intravenous injection, or intramuscular injection, but is not limited thereto.

In order to formulate into an injectable formulation, the composition of the present invention may be mixed with a stabilizer or buffer in water to prepare a solution or suspension, which may be formulated for unit administration in an ampoule or vial.

Alternatively, the composition of the present invention may be formulated into a variety of parenteral formulations, such as eye drops, microneedles, patches, and depots.

The composition of the present invention is administered in a pharmaceutically effective amount. In the present invention, "pharmaceutically effective amount" refers to an amount sufficient to treat a disease, and an effective dosage level may be determined according to factors including the type and severity of the patient's disease, the activity of the drug, the sensitivity to the drug, administration time, administration route, excretion rate, treatment period, and concurrently used drugs, and other factors well known in the medical field. The composition of the present invention may be administered as an individual therapeutic agent or in combination with other therapeutic agents, and may be administered sequentially or simultaneously with conventional therapeutic agents, and may be administered in a single dose or in multiple doses. In other words, the total effective amount of the composition of the present invention may be administered to a patient in a single dose, or may be administered by a fractionated treatment protocol in which multiple doses are administered over a long period of time. It is important to administer an amount that is able to achieve the maximum effect with the minimum amount without side effects by considering all of the above-described factors, and this may be easily determined by one of ordinary skill in the art.

The appropriate once-daily dose of enavogliflozin determined during clinical trials is 0.1 mg to 0.5 mg, and based on the results of the examples described below, it is expected that the enavogliflozin M1 metabolite may also be used with a dose of 0.1 mg to 0.5 mg similar to enavogliflozin.

When the pharmaceutical composition is formulated into a unit dosage form, the content of the active ingredient in the pharmaceutical composition may be 0.1 to 0.5 mg.

The pharmaceutical composition according to the present invention may be administered once to three times a day, for example, once a day, but is not limited thereto.

### [Advantageous Effects]

A pharmaceutical composition comprising the enavogliflozin M1 metabolite of the present invention as an active ingredient can be effectively used for the prevention or treatment of diabetes or heart failure.

### [Description of Drawings]

FIG. 1 shows graphs illustrating the SGLT1 activity inhibition effect of enavogliflozin, M1, M2, dapagliflozin, and empagliflozin on methyl α-D-glucopyranoside (AMG) uptake into Chinese hamster ovary (CHO)-SGLT1 cells.
FIG. 2 shows graphs illustrating the SGLT2 activity inhibition effect of enavogliflozin, M1, M2, dapagliflozin, and empagliflozin on AMG uptake into CHO-SGLT2 cells.
FIG. 3 shows graphs illustrating the change in the inhibitory effect of enavogliflozin, M1, M2, dapagliflozin, and empagliflozin on SGLT1 activity according to the pretreatment time of the drug.
FIG. 4 shows graphs illustrating the change in the inhibitory effect of enavogliflozin, M1, M2, dapagliflozin, and empagliflozin on SGLT2 activity according to the pretreatment time of the drug.
FIG. 5 shows the results of comparing the activity recovery ability of enavogliflozin, M1, M2, dapagliflozin, and empagliflozin for SGLT1.
FIG. 6 shows the results of comparing the activity recovery ability of enavogliflozin, M1, M2, dapagliflozin, and empagliflozin for SGLT2.
FIG. 7 shows the results of comparing the binding affinity and dissociation degree of enavogliflozin, M1, M2, and dapagliflozin for SGLT1.
FIG. 8 shows the results of comparing the binding affinity and dissociation degree of enavogliflozin, M1, M2, and dapagliflozin for SGLT2.
FIG. 9 shows a graph illustrating the change in blood glucose level over time after administration of enavogliflozin, M1, or M2 in normal rats.
FIG. 10 shows a graph illustrating the area under the blood glucose concentration-time curve (AUC) after administration of enavogliflozin, M1, or M2 in normal rats.
FIG. 11 shows a graph illustrating the non-fasting blood glucose levels observed up to 72 hours after administration of the test substance in streptozotocin (STZ)-induced type 1 diabetic rats.
FIG. 12 shows a graph illustrating the blood glucose level according to the oral glucose tolerance test in STZ-induced type 1 diabetic rats.
FIG. 13 shows a graph illustrating the area under the blood glucose concentration-time curve (AUC) according to the oral glucose tolerance test in STZ-induced type 1 diabetic rats.
FIG. 14 shows a graph illustrating the change in the level of inflammatory response-related cytokines over time after administration of the test substance in STZ-induced type 1 diabetic rats.

### [Modes of the Invention]

The advantages and features of the present invention and the method for achieving them will become clear with reference to the experimental examples and preparation examples described in detail below. However, the present invention is not limited to the experimental examples and preparation examples disclosed below, but may be implemented in various different forms, and the examples are provided only to ensure that the disclosure of the present invention is complete and to fully inform the scope of the invention to one of ordinary skill in the art to which the present invention pertains.

### [Examples]

### [Example 1] Evaluation of SGLT1 and SGLT2 inhibition of enavogliflozin metabolite

According to a preliminary study by the applicant of the present invention, it was confirmed that enavogliflozin (also referred to as DWP16001 in the following examples) showed higher inhibition of SGLT2 than SGLT1 compared to competing drugs dapagliflozin and ipragliflozin and exhibited no change in inhibition after pretreatment for up to two hours. The Dixon plot and Replot results for the inhibition evaluation according to the substrate concentration showed that the inhibition of SGLT2 exhibited a reversible competitive inhibition mechanism. However, when the intracellular drug concentration was quantified after culturing for 24 hours, it was confirmed that the amount of enavogliflozin remaining in the cells was significantly higher compared to dapagliflozin and ipragliflozin. In addition, in the case of enavogliflozin, the recovery ability for intracellular SGLT2 activity was delayed over time, but this did not occur in the competing drugs dapagliflozin and ipragliflozin. These results indicate that enavogliflozin may have better drug effect persistence than dapagliflozin and ipragliflozin.

The present inventors intended to evaluate whether the same results are observed for the M1 and M2 metabolites of enavogliflozin (herein referred to as M1 and M2, respectively). In addition, the present inventors intended to evaluate and compare dapagliflozin and empagliflozin, which have been actively undergoing clinical research.

### Example 1-1. Evaluation of inhibition activity of enavogliflozin metabolite on SGLT subtypes

### (1) Evaluation of SGLT1 inhibition

An SGLT1-overexpressing cell line (CHO-SGLT1 stable cells) and mock cells (CHO-mock cells) were cultured to reach 80% confluency, and then the cells were seeded in 96-well plates at 1x10⁵ cells/well.

After 24 hours, the medium was removed, and a pre-incubation buffer [10 mM hydroxyethylpiperazine ethane sulfonic acid (HEPES), 5 mM Tris, 140 mM choline chloride, 2 mM KCl, 1 mM CaCl₂, 1 mM MgCl₂, pH 7.4] was added, and the cells were cultured for one hour.

After one hour, the pre-incubation buffer was removed, and an incubation buffer [10 mM HEPES, 5 mM Tris, 140 mM NaCl, 2 mM KCl, 1 mM CaCl₂, 1 mM MgCl₂, pH 7.4] containing 10 µM [14C] methyl α-D-glucopyranoside (AMG), a substrate of SGLT1, and inhibitory evaluation substances at various concentrations was added and allowed to react in a thermo micro mixer at 37 °C for two hours.

The concentrations of inhibitory evaluation substances used in the experiment are described below.
Enavogliflozin: 1, 10, 100, 500, 1000, 5000, 20000, 50000 nM
M1: 1, 10, 100, 500, 1000, 5000, 20000, 50000 nM
M2: 1, 10, 100, 500, 1000, 5000, 20000, 50000 nM
Dapagliflozin: 1, 10, 100, 500, 1000, 5000, 20000, 50000 nM
Empagliflozin: 1, 10, 100, 500, 1000, 5000, 20000, 50000 nM

### For reference, the M2 metabolite has the compound name described below and the structure represented by Chemical Formula 2

### Compound name: (2S,3R,4R,5S,6R)-2-(7-chloro-6-(4-(1-hydroxycyclopropyl)benzyl)-2,3-dihydrobenzofuran-4-yl)-6-(hydroxymethyl)tetrahydro-2H-pyran-3,4,5-triol)

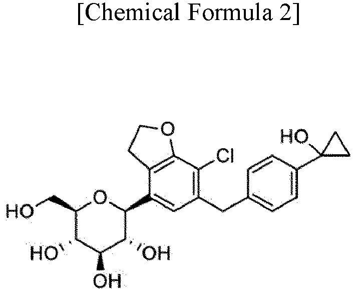

After a preset time, the cells were washed twice with 200 µL of ice-cold washing buffer [10 mM HEPES, 5 mM Tris, 140 mM choline chloride, 2 mM KCl, 1 mM CaCl₂, 1 mM MgCl₂, 100 mM AMG, pH 7.4].

After removing the medium, 40 µL of 10% sodium dodecyl sulfate (SDS) solution was added to lyse the cells, and then the samples were transferred to a measurement plate of a liquid scintillation counter, and 150 µL of OptiPhase Supermix^{®}, an isotope measurement cocktail solution, was added, and the cells were incubated overnight to ensure that the cell solution was homogeneously mixed with the cocktail solution.

[¹⁴C]AMG in the samples was measured using the liquid scintillation counter.

The intracellular uptake rate of [¹⁴C]AMG in the SGLT1-overexpressing cell line was compared with the uptake rate in the CHO-mock cells, and the IC₅₀ value with a 50% inhibition value (half maximal inhibitory concentration) was calculated from the graph of the intracellular uptake rate (% of control) of the substrate [¹⁴C]AMG and the concentration of the applied inhibitory evaluation substance. At this time, the inhibitory effect Sigmoid Eₘₐₓ model was used, and the WinNonlin program (ver. 2.0) was utilized. All data values were expressed as the mean ± standard deviation (SD) of three independent experiments.

### (2) Evaluation of SGLT2 inhibition

The evaluation was performed in the same manner as the evaluation of SGLT1 inhibition, except that an SGLT2-overexpressing cell line (CHO-SGLT2 stable cells) was used, and the inhibitory evaluation substances described below were used.

The concentrations of the inhibitory evaluation substances are described below.
Enavogliflozin: 0.01, 0.05, 0.2, 2, 10, 50, 100, 500 nM
M1: 0.01, 0.05, 0.2, 2, 10, 50, 100, 500 nM
M2: 0.01, 0.05, 0.2, 2, 10, 50, 100, 500 nM
Dapagliflozin: 0.01, 0.05, 0.2, 2, 10, 50, 100, 500 nM
Empagliflozin: 0.01, 0.05, 0.2, 2, 10, 50, 100, 500 nM

### (3) Evaluation of inhibition activity of enavogliflozin and M1 and M2 metabolites in SGLT1- and SGLT2-overexpressing cell lines

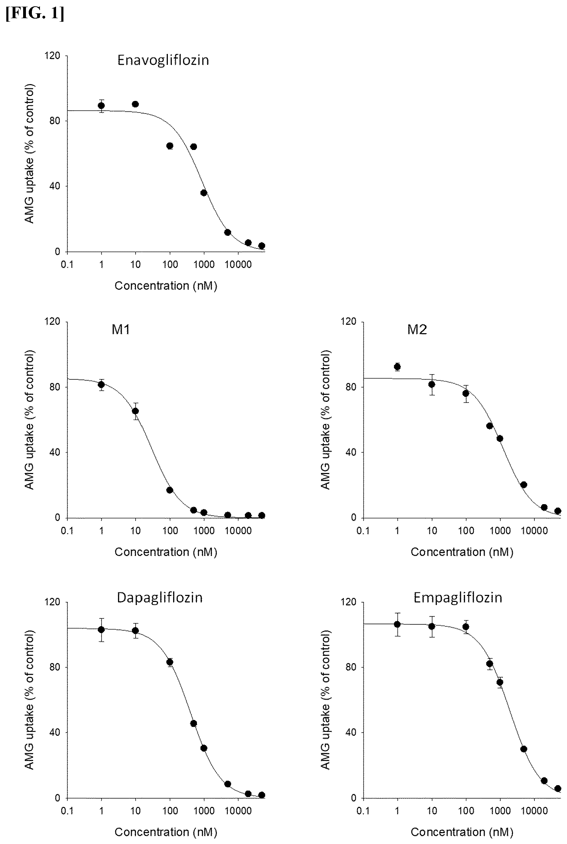

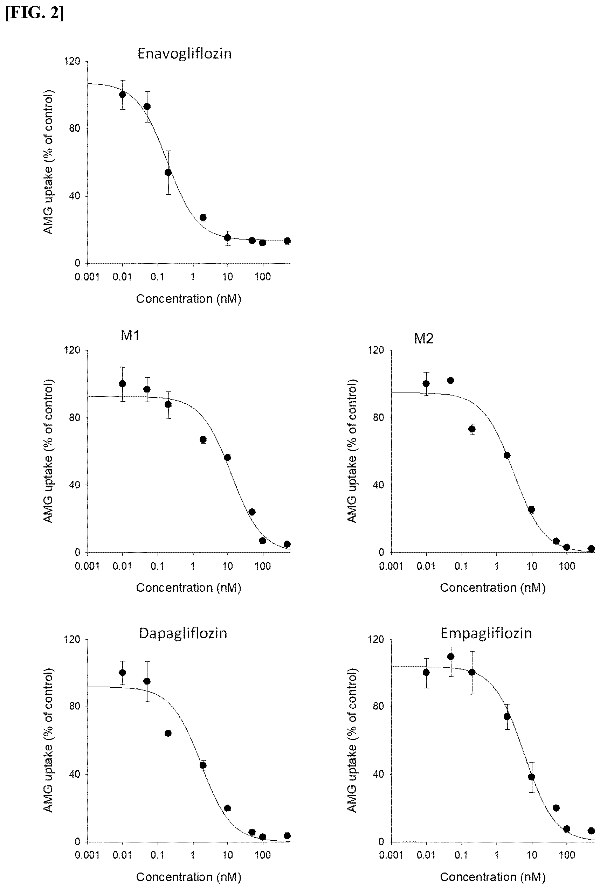

The inhibition activity of enavogliflozin, M1, M2, dapagliflozin, and empagliflozin on SGLT1 or SGLT2 was evaluated and shown in Table 1 below and FIGS. 1 and 2.

**[Table 1] Inhibitory effects of enavogliflozin, M1, M2, dapagliflozin, and empagliflozin on SGLT1 or SGLT2 activity**

| | IC₅₀ (nM) | | IC₅₀ ratio |
|---|---|---|---|
| | SGLT1 | SGLT2 | (SGLT1/SGLT2) |
| | mean ± SD (n=3) | mean ± SD (n=3) | |
| Enavogliflozin | 861 ± 59.4 | 0.34 ± 0.08 | 2538 |
| M1 | 28.4 ± 6.37 | 4.71 ± 0.69 | 6.0 |
| M2 | 1216 ± 165 | 3.02 ± 0.36 | 402 |
| Dapagliflozin | 403 ± 55.1 | 1.73 ± 0.15 | 233 |
| Empagliflozin | 1928 ± 76.0 | 6.65 ± 2.92 | 290 |

The inhibition constants derived from the evaluation results were similar to those of previous studies, and compared to dapagliflozin and empagliflozin, enavogliflozin was evaluated to have stronger inhibition of SGLT2 and better selectivity as determined by the ratio of the inhibition constants of SGLT1 and SGLT2. In addition, both enavogliflozin metabolites M1 and M2 exhibited inhibition of SGLT2.

Specifically, unlike enavogliflozin, M2, dapagliflozin, and empagliflozin, which selectively inhibit SGLT2, metabolite M1 showed an excellent SGLT1 activity inhibition effect (IC₅₀ value was about 28.4 nM), and also showed a very low value of the IC₅₀ ratio of SGLT1/SGLT2, confirming that it may serve as a dual inhibitor of both SGLT1 and SGLT2.

### Example 1-2. Evaluation of inhibition mechanism of enavogliflozin and M1 and M2 metabolites on SGLT1 and SGLT2

### (1) Comparative evaluation of the change in inhibition according to drug exposure time in SGLT1-overexpressing cell line

CHO-SGLT1 cells were pretreated with enavogliflozin, M1, M2, dapagliflozin, and empagliflozin at different concentrations in a sodium-free pretreatment buffer (Na negative pretreatment buffer) for one or two hours, and then the concentration-dependent inhibition of enavogliflozin on AMG uptake into CHO-SGLT1 cells was evaluated and compared with the group that was not pretreated. All data values are expressed as the mean ± SD of four independent experiments.

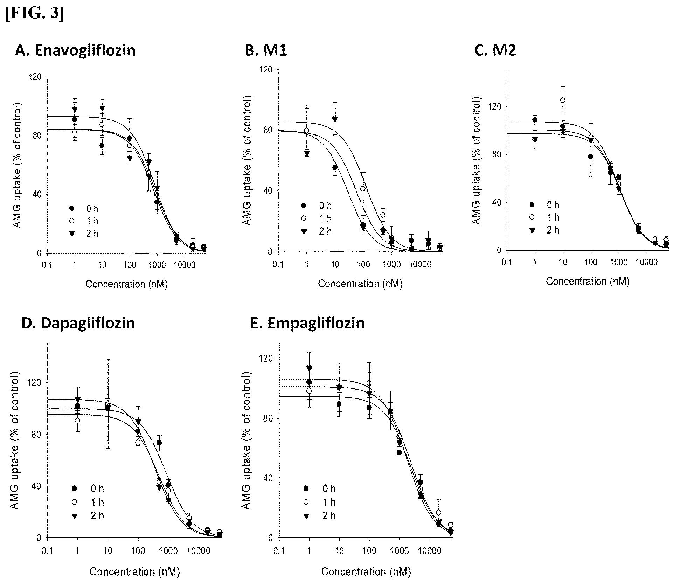

As a result of the evaluation, as can be seen in Table 2 and FIG. 3, enavogliflozin, M2, dapagliflozin, and empagliflozin did not show any significant change or increase in inhibition as the exposure time increased (p>0.05). On the other hand, the inhibition constant of M1 significantly increased as the exposure time increased (p<0.05).

**[Table 2] Changes in IC₅₀ (nM) values of enavogliflozin, M1, M2, dapagliflozin, and empagliflozin on SGLT1 activity according to pretreatment time**

| SGLT1 | IC₅₀ according to pretreatment time (nM) | | | P value |
|---|---|---|---|---|
| | 0 h | 1 h | 2h | |
| Enavogliflozin | 867 ± 163 | 889 ± 238 | 829 ± 194 | 0.972 |
| M1 | 29.2 ± 13.5 | 103 ± 22.4 | 76.6 ± 1.2 | 0.002 |
| M2 | 1079 ± 71 | 1022 ± 225 | 1179 ± 177 | 0.172 |
| Dapagliflozin | 808 ± 202 | 596 ± 363 | 434 ± 133 | 0.127 |
| Empagliflozin | 2431 ± 815 | 2355 ± 285 | 1738 ± 203 | 0.211 |

P value was assessed by one-way analysis of variance (ANOVA).

### (2) Evaluation of inhibition according to the concentration of substrate AMG in SGLT1-overexpressing cell line

In order to determine the inhibition mechanism and inhibition constant (Ki) for SGLT1, the concentration-dependent inhibition of enavogliflozin, M1, M2, dapagliflozin, and empagliflozin was evaluated by varying the concentration of substrate AMG in the CHO-SGLT1 cell line.

The uptake rate of AMG and the concentrations of enavogliflozin, M1, M2, dapagliflozin, and empagliflozin were plotted in a Dixon plot, and the AMG concentration and the Dixon slope were represented as a graph. In addition, the results were represented as an Lineweaver-Burk (L-B) plot, and the inhibitor concentrations and the L-B slope were represented as a graph (not shown). From these, the inhibition mechanism was confirmed, and the inhibition constant was calculated. The inhibition constant derived from the slope of the L-B plot and the inhibition mechanism are shown in Table 3.

**[Table 3] Ki values of enavogliflozin, M1, M2, dapagliflozin, and empagliflozin for SGLT1 activity**

| Compound | Ki (nM) | Inhibition mechanism |
|---|---|---|
| Enavogliflozin | 495 | Competitive inhibition |
| M1 | 84.8 | Competitive inhibition |
| M2 | 1872 | Competitive inhibition |
| Dapagliflozin | 502 | Competitive inhibition |
| Empagliflozin | 1708 | Competitive inhibition |

As can be seen in Table 3, the Ki value of the M1 metabolite for SGLT1 activity was 84.8 nM, which was the smallest value among the test substances. A smaller Ki value indicates higher affinity, and these results show that M1 has a higher affinity for SGLT1 than the test substances, and thus has a strong inhibition effect on SGLT1.

### (3) Comparative evaluation of the change in inhibition according to drug exposure time in SGLT2-overexpressing cell line

CHO-SGLT2 cells were pretreated with enavogliflozin, M1, M2, dapagliflozin, and empagliflozin at different concentrations in a sodium-free pretreatment buffer for one or two hours, and then the concentration-dependent inhibition of enavogliflozin on AMG uptake into CHO-SGLT2 cells was evaluated and compared with the group that was not pretreated. All data values are expressed as the mean ± SD of four independent experiments.

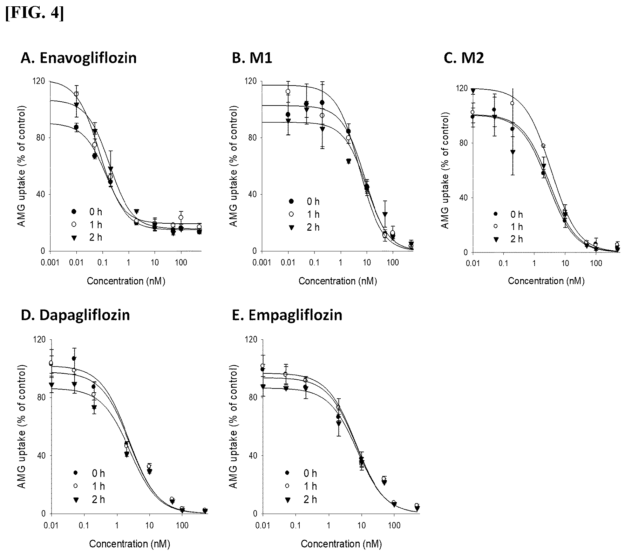

As a result of the evaluation, as can be seen in Table 4 and FIG. 4, the inhibition of enavogliflozin, M1, M2, dapagliflozin, and empagliflozin did not increase or significantly change even when the exposure time increased (p>0.05).

**[Table 4] Changes in IC₅₀ (nM) values of enavogliflozin, M1, M2, dapagliflozin, and empagliflozin on SGLT2 activity according to pretreatment time**

| SGLT2 | IC₅₀ according to pretreatment time (nM) | | | P value |
|---|---|---|---|---|
| | 0 h | 1 h | 2h | |
| Enavogliflozin | 0.388 ± 0.119 | 0.285 ± 0.188 | 0.519 ± 0.222 | 0.370 |
| M1 | 8.44 ± 0.77 | 5.24 ± 1.10 | 9.95 ± 3.14 | 0.321 |
| M2 | 2.75 ± 0.36 | 3.63 ± 0.83 | 3.24 ± 0.81 | 0.518 |
| Dapagliflozin | 2.35 ± 0.271 | 2.43 ± 0.220 | 2.52 ± 0.351 | 0.572 |
| Empagliflozin | 7.20 ± 2.33 | 6.67 ± 1.29 | 7.35 ± 0.82 | 0.645 |

P value was assessed by one-way ANOVA.

### (4) Evaluation of inhibition according to the concentration of substrate AMG in SGLT2-overexpressing cell line

In order to determine the inhibition mechanism and inhibition constant (Ki) for SGLT2, the concentration-dependent inhibition of enavogliflozin, M1, M2, dapagliflozin, and empagliflozin was evaluated by varying the concentration of substrate AMG in the CHO-SGLT2 cell line.

The uptake rate of AMG and the concentrations of enavogliflozin, M1, M2, dapagliflozin, and empagliflozin were plotted in a Dixon plot, and the AMG concentration and the Dixon slope were represented as a graph. In addition, the results were represented as an L-B plot, and the inhibitor concentrations and the L-B slope were represented as a graph (not shown). From these, the inhibition mechanism was confirmed, and the inhibition constant was calculated. The inhibition constant derived from the slope of the L-B plot and the inhibition mechanism are shown in Table 5.

**[Table 5] Ki values of enavogliflozin, M1, M2, dapagliflozin, and empagliflozin for SGLT2 activity**

| Compound | Ki (nM) | Inhibition mechanism |
|---|---|---|
| Enavogliflozin | 0.411 | Competitive inhibition |
| M1 | 7.49 | Competitive inhibition |
| M2 | 6.18 | Competitive inhibition |
| Dapagliflozin | 1.23 | Competitive inhibition |
| Empagliflozin | 5.15 | Competitive inhibition |

### Example 1-3. Comparative evaluation of activity recovery ability for SGLT1 and SGLT2 after 24-hour pretreatment with enavogliflozin and metabolites

### (1) Comparative evaluation of activity recovery ability after washout of enavogliflozin and metabolites in SGLT1-overexpressing cell line

When an SGLT1-overexpressing cell line (CHO-SGLT1 stable cells) was cultured to reach 80% confluency, cells were seeded at 1x10⁵ cells/well in a 96-well plate. After 24 hours, the medium was removed and replaced with a medium containing the evaluation substances at different concentrations, and the cells were cultured for 24 hours.

The concentrations of the evaluation substances used in the experiment are described below.
Enavogliflozin: 50, 500, 5000, 20000 nM
M1: 50, 500, 5000, 20000 nM
M2: 50, 500, 5000, 20000 nM
Dapagliflozin: 50, 500, 5000, 20000 nM
Empagliflozin: 50, 500, 5000, 20000 nM

A pre-incubation buffer [10 mM HEPES, 5 mM Tris, 140 mM choline chloride, 2 mM KCl, 1 mM CaCl₂, 1 mM MgCl₂, pH 7.4] without the test substance was added to the SGLT1-overexpressing cell line treated with the drug for 24 hours and incubated for 1, 2, 4, 8, and 24 hours to dissociate the drug bound to SGLT1.

After one hour, the pre-incubation buffer was removed and an incubation buffer [10 mM HEPES, 5 mM Tris, 140 mM NaCl, 2 mM KCl, 1 mM CaCl₂, 1 mM MgCl₂, pH 7.4] containing 10 µM [¹⁴C]AMG, a substrate of SGLT1, was added and allowed to react for two hours at 37 °C in a thermo micro mixer. After two hours, the cells were washed twice with 200 µL of ice-cold washing buffer [10 mM HEPES, 5 mM Tris, 140 mM choline chloride, 2 mM KCl, 1 mM CaCl₂, 1 mM MgCl₂, 100 mM AMG, pH 7.4].

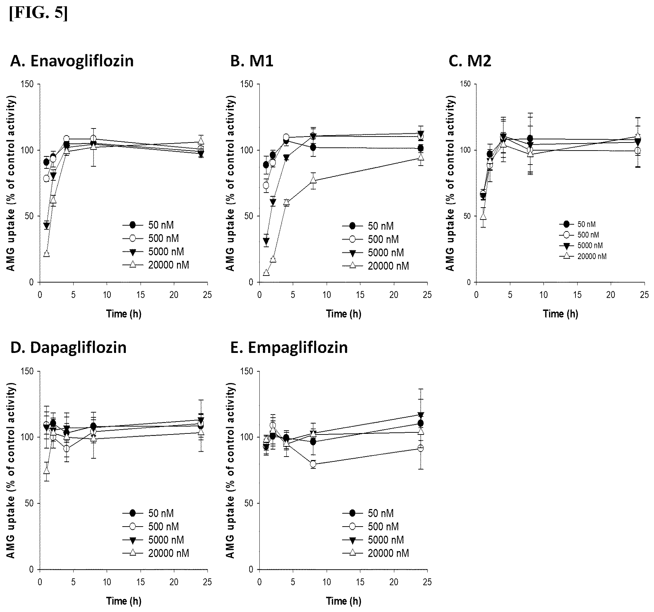

After removing the medium, 40 µL of 10% SDS solution was added to lyse the cells, and then the samples were transferred to a measurement plate of a liquid scintillation counter, and 150 µL of OptiPhase Supermix^{®}, an isotope measurement cocktail solution, was added, and the cells were incubated overnight to ensure that the cell solution was homogeneously mixed with the cocktail solution. [¹⁴C]AMG in the samples was measured using the liquid scintillation counter, and the intracellular uptake rate (% of control) of the substrate [14C]AMG in the SGLT1-overexpressing cell line is shown in FIG. 5. All data values were expressed as the mean ± SD of four independent experiments.

As can be confirmed in FIG. 5, in the activity of SGLT1 decreased when the CHO-SGLT1 cell line was treated with enavogliflozin, M1, M2, dapagliflozin, or empagliflozin for 24 hours, the recovery ability for SGLT1 activity showed a difference depending on the time when the drug was removed from the medium.

In other words, when the cells were treated with enavogliflozin, the activity incompletely recovered for up to two hours after washout, but recovered after four hours (FIG. 5A).

In the case of M1 treatment, the activity partially recovered for up to two hours after washout at low concentrations (50, 500, 5000 nM), but in the 20,000 nM treatment group, activity recovery was incomplete even 24 hours after washout (FIG. 5B).

In the case of M2 treatment, a trend that was very similar to enavogliflozin was observed. In other words, the activity incompletely recovered for up to two hours after washout, but recovered after four hours (FIG. 5C).

In the case of dapagliflozin or empagliflozin treatment, the activity completely recovered after two hours after washout, and it was confirmed that recovery was faster at the same concentrations than in the case of enavogliflozin (FIGS. 5D and 5E).

From the above-described results, it was confirmed that the dissociation of M1 from SGLT1 was the slowest, the dissociation of enavogliflozin and M2 was fast, and the dissociation of dapagliflozin and empagliflozin was fast. The above-described results mean that among the evaluated substances, M1 binds to SGLT1 the longest.

### (2) Comparative evaluation of activity recovery ability after washout of enavogliflozin and metabolites in SGLT2-overexpressing cell line

The evaluation method was the same as that for SGLT1-overexpressing cells, except that an SGLT2-overexpressing cell line (CHO-SGLT2 stable cells) was used and the concentrations of the evaluation substances used are as described below.
Enavogliflozin: 0.5, 5, 50, 500 nM
M1: 0.5, 5, 50, 500 nM
M2: 0.5, 5, 50, 500 nM
Dapagliflozin: 0.5, 5, 50, 500 nM
Empagliflozin: 0.5, 5, 50, 500 nM

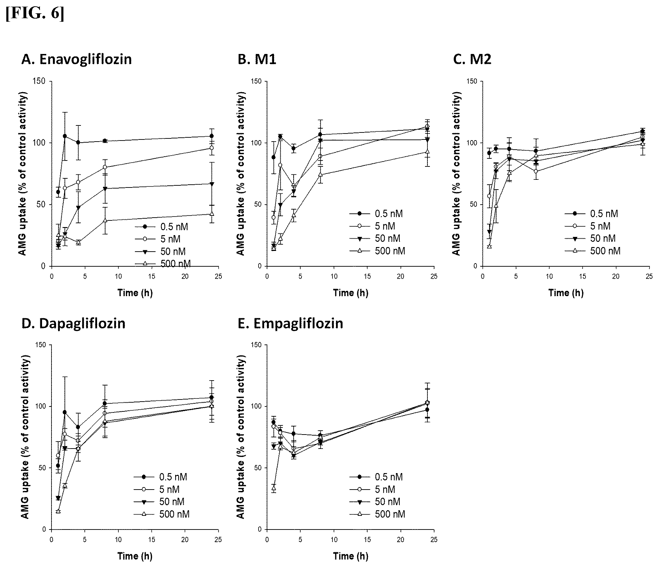

As can be confirmed in FIG. 6, in the activity of SGLT2 decreased when the CHO-SGLT2 cells were treated with enavogliflozin, M1, M2, dapagliflozin, or empagliflozin for 24 hours, the recovery ability for SGLT2 activity showed a difference depending on the time of drug removal from the medium.

In the case of enavogliflozin treatment, the recovery of activity over time after washout showed different patterns depending on the concentration of enavogliflozin. When the cells were treated with 0.5 nM, the activity recovered after two hours, but when treated with 5 nM, recovery was shown after 24 hours. When the cells were treated with 50 and 500 nM, activity recovery was unstable even after 24 hours, showing less than 50% recovery. The above-described results indicate that the recovery of activity was slow because the intracellular concentration of enavogliflozin was maintained or dissociation from SGLT2 was delayed (FIG. 6A).

In the case of M1 treatment, the activity recovered after two hours at low concentration (0.5 nM), but when the cells were treated with 5 and 50 nM and washed out, the activity recovered over time for up to eight hours and almost recovered after 24 hours. When the cells were treated with 500 nM, it was confirmed that the recovery of activity was delayed (FIG. 6B).

In the case of M2 treatment, it was confirmed that the difference according to the concentration was not significant, and the activity recovered over time. In other words, the activity recovered over time for up to four hours after washout, and recovered after eight hours (FIG. 6C).

In the case of dapagliflozin treatment, a trend that was similar to M2 was shown. In other words, it was confirmed that the difference according to the concentration was not significant, and the activity recovered over time. The activity recovered over time for up to four hours after washout, and recovered after 8 hours (FIG. 6D).

In the case of empagliflozin treatment, it was confirmed that the activity completely recovered two hours after washout, and the recovery was faster at the same concentrations than in the case of enavogliflozin, M1, M2, and dapagliflozin (FIG. 6E).

The above-described results show that enavogliflozin dissociated the slowest from SGLT2, and in case of high concentrations, recovery of activity was incomplete even after 24 hours, showing less than 50% recovery. In the case of M1, it was confirmed that recovery of activity was delayed depending on the concentration and washout time. In M2 and dapagliflozin, the recovery of activity did not show a large difference according to the concentration and showed a delay depending on the washout time, and empagliflozin showed the fastest activity recovery without a delay according to the pretreatment concentration or time.

### [Example 2] Evaluation of drug-target binding affinity and dissociation degree of enavogliflozin metabolites

When SGLT1- and SGLT2-overexpressing cell lines (CHO-SGLT1, CHO-SGLT2 stable cells) were cultured to reach 60% confluency, the cells were treated with 0.05% trypsin to detach the cells from the bottom of the plate, suspended in 200 µL of culture medium, and used at room temperature.

To evaluate the target binding affinity and dissociation degree of enavogliflozin, M1, M2, and dapagliflozin for SGLT1 and SGLT2, glucose currents were recorded using a whole-cell mode patch clamp, and the drug-target binding affinity and dissociation degree were confirmed by evaluating how long they were bound to the target, through washout.

Glucose reabsorption is induced by the Na⁺ electrochemical potential gradient in the kidneys via SGLT2, and glucose diffuses across the cell membrane into the blood via glucose transporter 2 (GLUT-2), which enables facilitated glucose transport. While one Na⁺ ion is absorbed per glucose molecule through SGLT2, two Na⁺ ions are absorbed per glucose molecule through SGLT1. Therefore, by varying the composition of the buffer, only the glucose current was recorded, excluding the Na⁺ current.

The experiment was conducted as described below.
1) Using a Narishige puller, a glass micropipette was made in two stages of 62.7 °C and 57 °C, filled with an internal solution (145 mM CsCl, 5 mM NaCl, 10 mM HEPES, 11 mM ethylene glycol-bis(2-aminoethylether)-N,N,N',N'-tetraacetic acid (EGTA), pH 7.2), and then inserted into the Ag/AgCl₂ electrode of the patch clamp head-stage.
2) SGLT1 or SGLT2 cells were seeded on a chamber which was coated with poly-L-lysine in advance, and then an external solution (150 mM NaCl, 10 mM HEPES, 1 mM CaCl₂, 1 mM MgCl₂, pH 7.4) was flowed to ensure that the cells were sufficiently submerged. A temperature control device was used to maintain the temperature of the chamber at 36.5 ± 0.5 °C.
3) Using a micromanipulator, the glass micropipette was attached to the cell membrane to create a giga-ohm seal, which was then ruptured to detach the cell membrane (whole-cell mode, 3 to 5 MΩ).
4) The cells were fixed at -60 mV (holding potential), and the current [I^{total} current (Na⁺/Glucose current (pA))] was measured in a gap-free data acquisition mode.
5) The external solution was continuously flowed into the chamber containing the cells, and the current (I^{total}) was recorded for about 30 seconds to 1 minute (stabilization), and then the external solution with added glucose (Na⁺/Glucose external) (150 mM NaCl, 10 mM HEPES, 1 mM CaCl₂, 1 mM MgCl₂, 100 mM glucose, pH 7.4) was flowed to record the glucose current (I^{glucose}). Then, the cells were exposed to the external solution with added glucose containing one concentration of the compound for two minutes, and the glucose current suppressed by the compound was recorded.
6) After confirming the suppressed current, the external solution was replaced with a new glucose-containing solution that did not contain the compound, and the change in the current was confirmed while washing out, and recorded for a minimum of 3 minutes and a maximum of 10 minutes (when the reaction continued longer and the seal could be maintained, the current was recorded for up to 15 minutes).
7) After confirming the recovery of the current, the solution was replaced with a sodium-free external solution (Na⁺-free external) (150 mM Choline-Cl, 1 mM CaCl₂, 1 mM MgCl₂, pH 7.4) and the Na⁺ current was recorded. After the current was stabilized, the solution was replaced with the external solution to record the current in the resting state when the cells were not stimulated, thereby securing the reliability of the experimental system.

For statistical analysis of data, pCLAMP10.4 (Molecular Devices, LLC, San Jose, CA, USA) or Origin6.0 (OriginLab, Northampton, MA, USA) was used.

The test results were expressed as the mean ± SD of the mean. Statistical significance was verified using the nonparametric Kruskal-Wallis test using Statistical Package for the Social Sciences (SPSS) for Windows (version 26.0, IBM Corp., Armonk, NY, USA). The significance level was set at p<0.05.

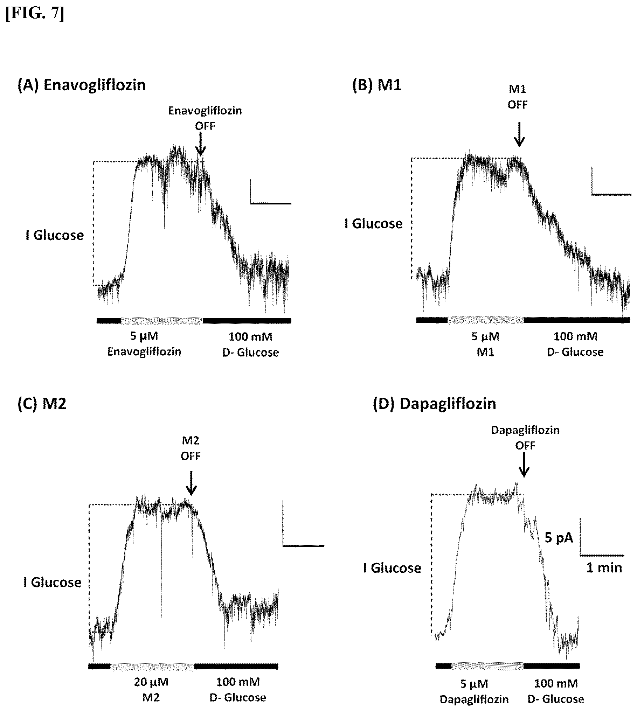

The results of evaluating the binding affinity and dissociation of enavogliflozin, M1, M2, and dapagliflozin for SGLT1 are shown in FIG. 7 and Table 6.

It was confirmed that the half-time of recovery (T_{1/2,off}) of enavogliflozin for SGLT1 was 34.9 seconds, and M2 (23.5 seconds) and dapagliflozin (30.5 seconds) dissociated at similar rates. On the other hand, it was confirmed that the half-time of recovery (T_{1/2,off}) of M1 was 59.8 seconds, indicating that M1 dissociated 1.7 times slower than enavogliflozin and 1.96 times slower than dapagliflozin.

These results suggest that M1 has an effect on binding to the SGLT1 protein and maintaining the bond thereto longer than enavogliflozin and its competitor dapagliflozin, and that efficacy may be maintained.

**[Table 6] Comparison of binding affinity and dissociation degree of enavogliflozin, M1, M2, and dapagliflozin for SGLT1**

| Compound | Treatment concentration | Binding affinity | | Dissociation degree | |
|---|---|---|---|---|---|
| | | T_{1/2,on} (s) | n | T_{1/2,off} (s) | n |
| Enavogliflozin | 5 µM | 11.5 ± 4.2 | 4 | 34.9 ± 9.6 | 4 |
| M1 | 5 µM | 18.8 ± 4.1 | 5 | 59.8 ± 11.5^{*,+} | 5 |
| M2 | 20 µM | 15.7 ± 6.5 | 4 | 23.5 ± 10.4 | 4 |
| Dapagliflozin | 5 µM | 15.7 ± 8.8 | 4 | 30.5 ± 9.1 | 4 |

Each data point represents the mean ± SD (n = 4 - 5); T_{1/2,on}: half-time on rate; T_{1/2,off}: half-time off rate; *: p < 0.05 compared to M2, +: p < 0.05 compared with dapagliflozin by post-hoc analysis followed by the significant nonparametric Kruskal-Wallis test.

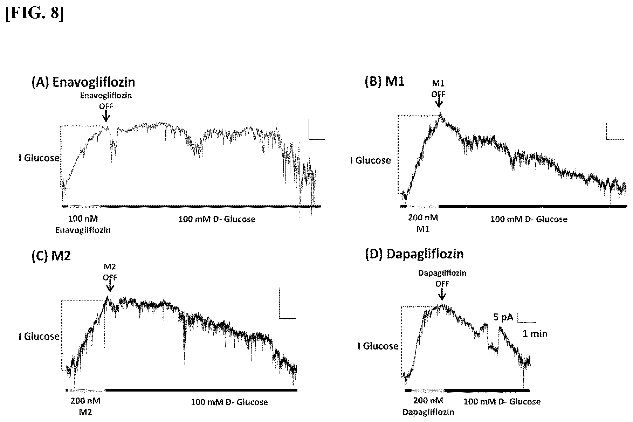

In addition, the results of evaluating the binding affinity and dissociation degree of enavogliflozin, M1, M2, and dapagliflozin for SGLT2 are shown in FIG. 8 and Table 7.

The half-time of recovery (T_{1/2,off}) of enavogliflozin for SGLT2 was 745.5 seconds, showing the slowest dissociation rate among the evaluated substances. It was about 2.8 times slower than the competing substance dapagliflozin (265.2 seconds), and M1 (369.9 seconds) and M2 (540.4 seconds) showed dissociation rates that were 2 times and 1.38 times slower than enavogliflozin, respectively.

These results suggest that enavogliflozin binds to the SGLT2 protein the longest, and that the inhibition effect may be maintained as the bond is retained. In addition, M1 and M2 may also maintain efficacy because they bind to the SGLT2 protein and dissociate more slowly than their competitor, dapagliflozin.

In conclusion, similar to the SGLT-1/SGLT-2 dual inhibitory effect of M1, the recovery of inhibitory activity upon wash-out from the SGLT-1 target showed a slower dissociation time than M2. In addition, it was found that M2 maintained binding to the SGLT-2 target longer than M1.

**[Table 7] Comparison of binding affinity and dissociation degree of enavogliflozin, M1, M2, and dapagliflozin for SGLT2**

| Compound | Treatment concentration | Binding affinity | | Dissociation degree | |
|---|---|---|---|---|---|
| | | T_{1/2,on} (s) | n | T_{1/2,off} (s) | n |
| Enavogliflozin | 100 µM | 29.5 ± 5.5 | 6 | 745.5^{+,#} | 4 |
| M1 | 200 µM | 52.5 ± 3.8^{*,+} | 3 | 369.9 ± 56.5 | 3 |
| M2 | 200 µM | 51.8 ± 10.5^{*,+} | 3 | 540.4 ± 217.0^{+,#} | 3 |
| Dapagliflozin | 200 µM | 31.2 ± 4.7 | 5 | 265.2 ± 143.9 | 4 |

Each data point represents the mean ± SD (n = 3 - 6); T_{1/2,on}: half-time on rate; T_{1/2,off}: half-time off rate; *: p < 0.05 compared to enavogliflozin, +: p < 0.05 compared with dapagliflozin, #: p < 0.05 compared with empagliflozin by post hoc analysis followed by the significant nonparametric Kruskal-Wallis test.

### [Example 3] Evaluation of antidiabetic efficacy by intravenous administration of enavogliflozin metabolite

Enavogliflozin, M1, or M2 was intravenously administered once to normal rats, and antidiabetic efficacy was evaluated.

To perform the oral glucose tolerance test (OGTT) and examine urinary glucose excretion (UGE), enavogliflozin, M1, or M2 was administered intravenously to male 8-week-old normal rats at a dose of 1 mg/kg, and then a glucose solution (2 g/kg) was administered to load blood sugar. The blood sugar level was measured before glucose solution administration and at 5, 10, 15, 20, 30, 40, 60, 90, and 120 minutes after the administration to evaluate oral glucose tolerance. In addition, the UGE was evaluated by measuring the amount of glucose excreted in the urine 6, 24, 48, and 72 hours after the administration of the glucose solution.

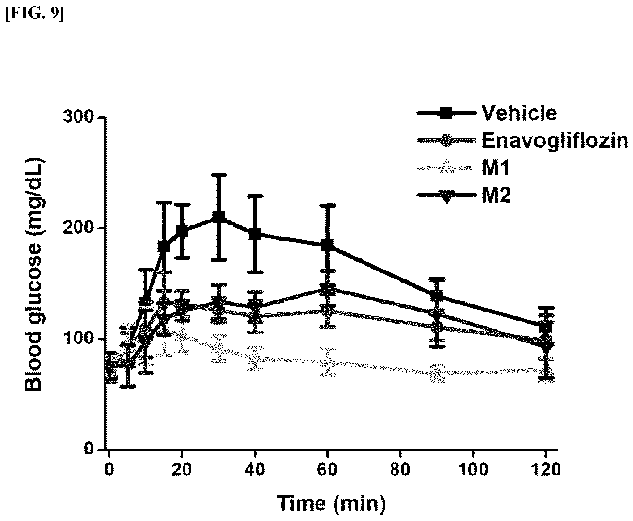

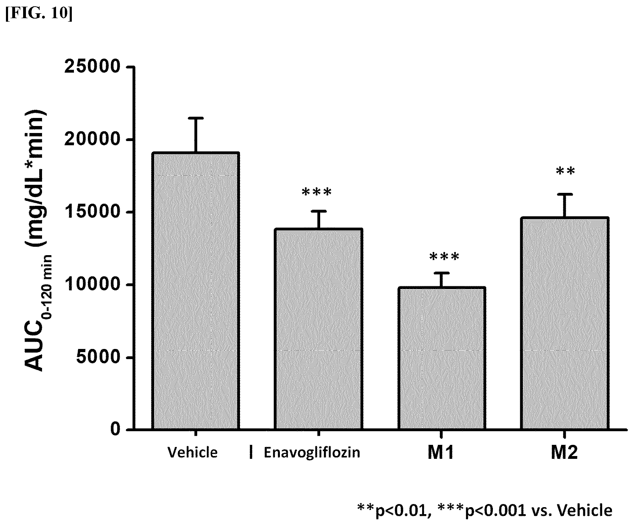

FIG. 9 shows a graph illustrating the change in blood glucose level over time after intravenous administration of each test substance at a dose of 1 mg/kg to normal rats. FIG. 10 shows a graph illustrating the analysis of the area under the blood glucose concentration-time curve (AUC) for 120 minutes after administration of the test substance.

As can be seen in FIGS. 9 and 10, it was confirmed that enavogliflozin, M1, and M2 improved glucose tolerance when intravenously administered to normal rats at a dose of 1 mg/kg. Metabolite M1 showed superior glucose tolerance to enavogliflozin when administered intravenously, and a similar pattern to enavogliflozin was observed for M2.

When compared in terms of blood glucose AUC, M1 showed the most significant decrease in blood glucose AUC, and enavogliflozin and M2 showed lower decrease rates than M1. The glucose tolerance inhibition rate was improved by 27% for enavogliflozin, 49% for M1, and 23% for M2 compared to the vehicle (FIG. 10).

Meanwhile, when UGE was confirmed, UGE was observed from six hours after intravenous administration of 1 mg/kg for metabolites M1 and M2. The amount and pattern of glucose excretion of M1 and enavogliflozin were similar, but in the case of M2, less glucose was excreted than enavogliflozin and M1 for up to 24 hours.

In summary of the above-described results, it is judged that intravenous administration of the test drug exhibits *in vivo* activity by acting on SGLT1 and SGLT2 in the kidneys.

Since the IC₅₀ of metabolite M1 for SGLT2 is higher than that of enavogliflozin, but metabolite M1 shows a stronger effect on SGLT1, it is judged that metabolite M1 serves as a dual inhibitor for SGLT1 and SGLT2, and ultimately, M1 exhibits antidiabetic efficacy similar to enavogliflozin.

M2 exhibited an effect for up to 24 hours and showed a weaker effect than that of M1, which is presumed to be because 1) the IC₅₀ of M2 for SGLT1 was higher than that of M1, and 2) rapid dissipation occurred (confirmed by T_{1/2}=0.8 h).

### [Example 4] Evaluation of antidiabetic efficacy of enavogliflozin metabolite in type 1 diabetic rat model

To evaluate the antidiabetic efficacy of the enavogliflozin metabolites in a streptozotocin (STZ)-induced type 1 diabetic rat model, test groups were set up as shown in Table 8, and experiments were conducted.

**[Table 8]**

| Group | Substance | Dose | Number of rats (n) | Regimen | Solvent |
|---|---|---|---|---|---|
| G1 | Saline solution (normal control) | - | 6 | - | |
| G2 | STZ (vehicle) | - | 10 | Single-dose administration by intravenous injection | 30% PEG-400 |
| G3 | M1 | 0.01 mg/kg | 10 | | |
| G4 | | 0.1 mg/kg | 10 | | |
| G5 | | 1 mg/kg | 10 | | |
| G6 | Enavogliflozin | 1 mg/kg | 10 | | |
| G7 | Sotagliflozin | 1 mg/kg | 10 | | |

STZ was administered to Sprague Dawley (SD) male 5-week-old rats on day 0 (G2 to G7, excluding G1), and non-fasting blood glucose levels were measured on day 3 to determine whether type 1 diabetes was induced. On day 7, the experimental substances were administered to each group, and blood and urine samples were collected at 6 h, 24 h, 48 h, and 72 h to measure non-fasting blood glucose levels. On day 14 of the experiment, the experimental animals were fasted for 16 h, and on day 15, 2 g/kg of glucose solution was orally administered after the experimental substances were administered. Thereafter, blood glucose levels were measured at 15, 30, 60, 90, and 120 minutes after glucose administration. After blood collection, the serum was separated, and the IL-6 inflammatory response was evaluated using an interleukin-6 (IL-6) enzyme-linked immunosorbent assay (ELISA) kit.

### (1) Non-fasting blood sugar level

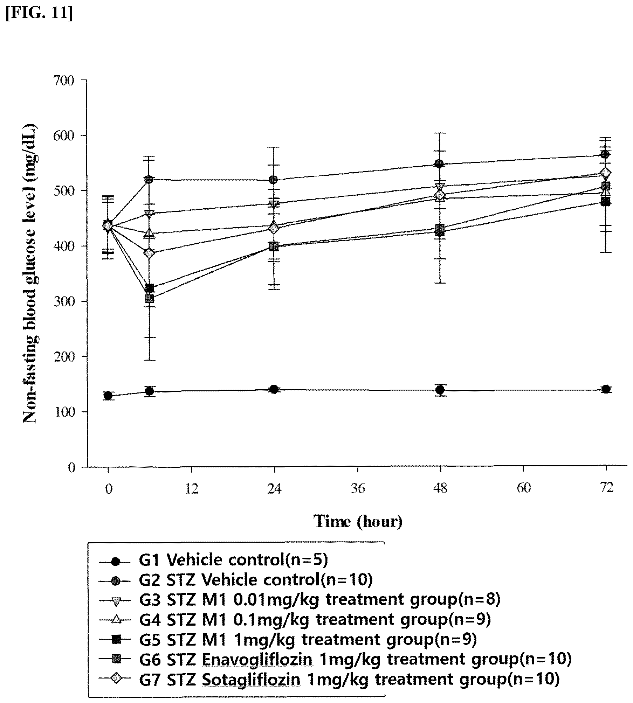

FIG. 11 shows the non-fasting blood sugar levels measured at 6, 24, 48, and 72 hours after the first administration of the test drug to an STZ-induced type 1 diabetic rat model.

As can be seen in FIG. 11, it was observed that blood sugar level was maintained low in G5 (enavogliflozin M1 metabolite) and G6 (enavogliflozin) at the same dose of 1 mg/kg, and it was confirmed that a statistically significant blood sugar-lowering effect lasted for up to 48 hours after administration compared to the vehicle group. In particular, G5 exhibited the lowest blood sugar level at 48 hours after administration, indicating a continuous blood sugar-lowering effect.

Compared to G7, which was the group administered 1 mg/kg of sotagliflozin approved for type 1 diabetes, G5 and G6 at the same dose showed a better blood sugar-lowering effect.

### (2) Oral glucose tolerance

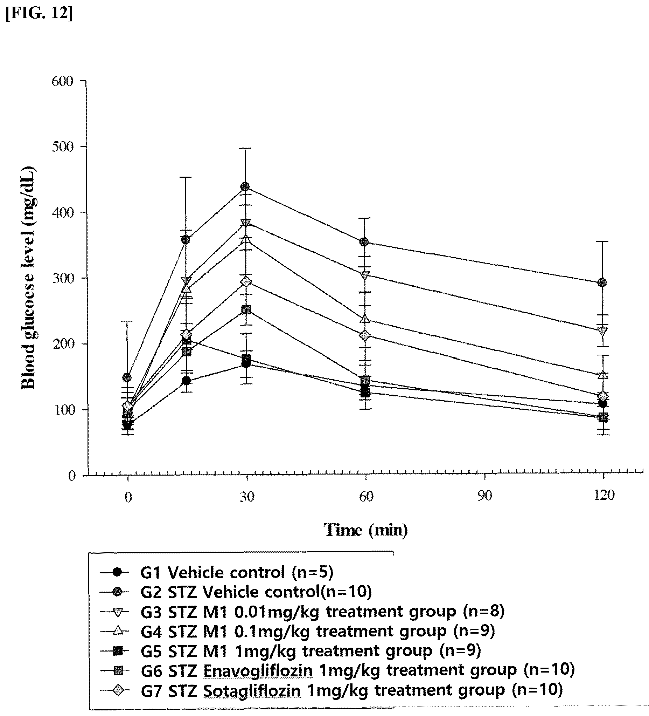

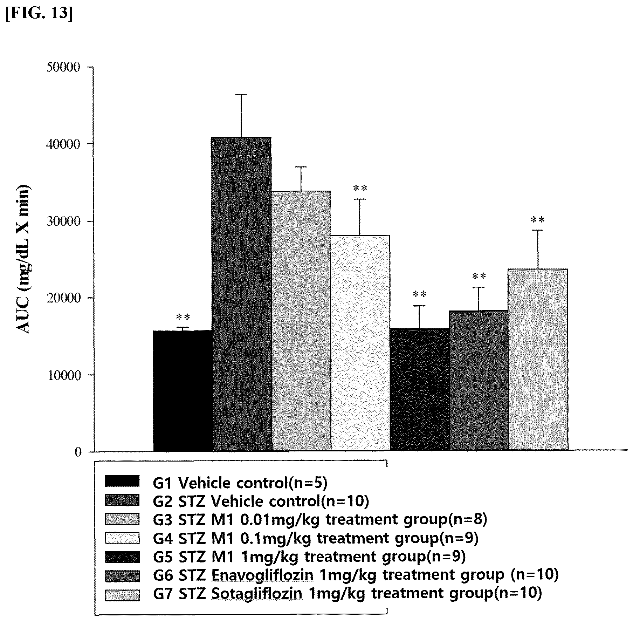

FIGS. 12 and 13 show the blood glucose levels measured before glucose administration (0 min) and at 15, 30, 60, and 120 minutes after glucose administration and the calculated AUC.

As can be seen in FIG. 12, lowering of the blood glucose level was observed in G5, which was administered 1 mg/kg of the enavogliflozin M1 metabolite, G6, which was administered 1 mg/kg of enavogliflozin, and G7, which was administered 1 mg/kg of sotagliflozin, and in the case of M1 metabolite, dose-dependent improvement in glucose tolerance was confirmed. In particular, the G5 group, which was administered 1 mg/kg of the enavogliflozin M1 metabolite, exhibited the fastest and strongest blood glucose lowering effect, and the effect was much superior to that of the G7 group, which was administered 1 mg/kg of sotagliflozin.

As can be seen in FIG. 13, the G5 group administered 1 mg/kg of enavogliflozin M1 metabolite showed the best oral glucose tolerance improvement effect in terms of the AUC value, and G5 showed an AUC value similar to that of G1, which was the control group administered the vehicle, confirming the superior blood sugar controlling effect of the enavogliflozin M1 metabolite in the OGTT.

### (3) Evaluation of inflammatory responses

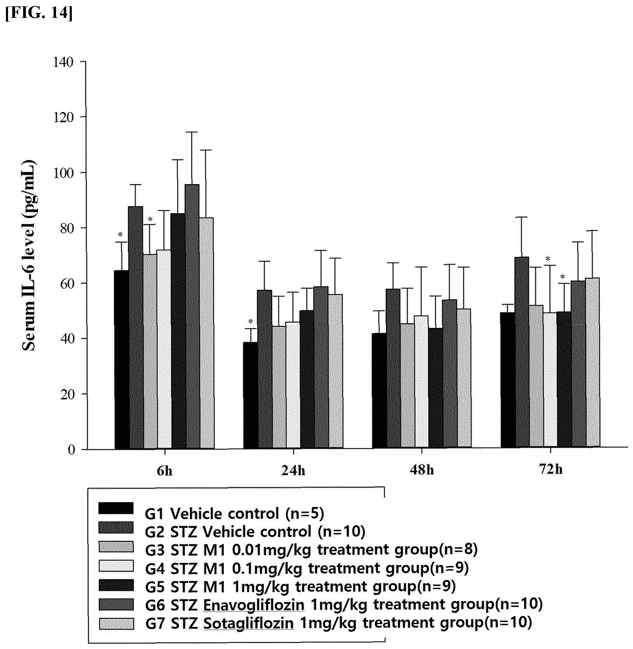

FIG. 14 shows the results of measuring cytokines after administering test drugs to an STZ-induced type 1 diabetic rat model.

As can be seen in FIG. 14, it was confirmed that interleukin-6 (IL-6), a cytokine that regulates the inflammatory response in the vehicle group, increased at all time points (6, 24, 48, 72 hours) and was reduced by M1 administration. Compared to the slight decrease in IL-6 in the enavogliflozin and sotagliflozin administration groups, M1 showed a significant decrease in all dose groups (0.01, 0.1, 1 mg/kg), and in particular, it showed a more significant decrease trend in the 0.01 mg/kg group after 6 hours, and in the 0.1 mg/kg and 1 mg/kg groups after 72 hours.

These results suggest that M1 can regulate the inflammatory response accompanying type 1 diabetes.

## Claims

1. A pharmaceutical composition for use in preventing or treating diabetes or heart failure, comprising an enavogliflozin M1 metabolite represented by Chemical Formula 1 below or a pharmaceutically acceptable salt thereof as an active ingredient.

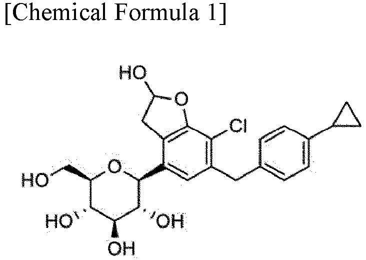

2. The pharmaceutical composition of claim 1, wherein the diabetes is type 1 diabetes.

3. The pharmaceutical composition of claim 1, wherein the diabetes is type 2 diabetes.

4. The pharmaceutical composition of claim 1, wherein the pharmaceutical composition is for oral or parenteral administration.

5. The pharmaceutical composition of claim 1, wherein a once-daily dose of the enavogliflozin M1 metabolite of Chemical Formula 1 or a pharmaceutically acceptable salt thereof is 0.1 to 0.5 mg.

6. The pharmaceutical composition of claim 1, wherein the pharmaceutical composition is administered once a day.

7. A sodium glucose cotransporter 1 (SGLT1)/sodium glucose cotransporter 2 (SGLT2) dual inhibitor comprising an enavogliflozin M1 metabolite represented by Chemical Formula 1 below or a pharmaceutically acceptable salt thereof as an active ingredient.
